(19) Europäisches Patentamt European Patent Office Office européen des brevets

(11) **EP 2 363 196 B1**

(12) **EUROPEAN PATENT SPECIFICATION**

(45) Date of publication and mention of the grant of the patent:
**12.07.2017 Bulletin 2017/28**

(51) Int Cl.:
***A61M 1/34*** *(2006.01)*   ***B01D 63/02*** *(2006.01)*

(21) Application number: **10155351.9**

(22) Date of filing: **03.03.2010**

(54) **DIFFUSION AND/OR FILTRATION DEVICE**

**DIFFUSIONS- UND/ODER FILTRATIONSVORRICHTUNG**

**DISPOSITIF DE DIFFUSION ET/OU FILTRATION**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**07.09.2011 Bulletin 2011/36**

(73) Proprietor: **Gambro Lundia AB**
**220 10 Lund (SE)**

(72) Inventors:
• **Drieschmanns, Hans-Rainer**
**72072, Tübingen (DE)**
• **Wittner, Bernd**
**21136, Malmö (SE)**
• **Beck, Werner**
**72108, Rottenburg (DE)**
• **Hildwein, Helmut**
**72189, Vöhringen (DE)**

(74) Representative: **Perchenek, Nils**
**Gambro Dialysatoren GmbH**
**Legal and Intellectual Property**
**Holger Crafoord-Strasse 26**
**72379 Hechingen (DE)**

(56) References cited:
**EP-A1- 1 201 293**   **WO-A1-01/66237**
**WO-A1-98/28064**   **WO-A1-98/33581**

**Description**

**Technical Field**

The present disclosure relates to a diffusion and/or filtration device comprising at least two different types of hollow fiber membranes. The device can be used in hemodialysis, hemodiafiltration and hemofiltration.

**Description of the Related Art**

Diffusion and/or filtration devices comprising hollow fiber membranes are used in various medical treatments which remove unwanted substances from body fluids, e.g., blood. Examples of such treatments are hemodialysis, hemodiafiltration and hemofiltration.

The filtration devices generally encompass a casing comprising a tubular section with end caps capping the mouths of the tubular section. A bundle of hollow fiber membranes is usually arranged in the casing in a way that a seal is provided between the first flow space formed by the fiber cavities and a second flow space surrounding the membranes on the outside. The first flow space is sealed off by end caps placed on the tubular section which provide inflow/outflow access to the first space. The tubular section generally comprises means for inflow/outflow access to the second space.

The person skilled in the art can chose from various types of hollow fiber membranes having different separation properties, according to the separation characteristics desired for the device. The individual types of membranes can differ in chemical composition; surface properties like charge, coatings, hydrophilicity/hydrophobicity, functional groups; membrane structure; or dimensions, e.g., length, diameter, wall strength, pore size of the fibers. These differences give rise to differences in the separation properties of the individual membrane types.

In general, hollow fiber membranes are specifically developed for the intended application, and their separation properties are designed to remove specific substances or substances within a certain molecular weight range. If a device having different separation characteristics is required, another type of membrane has to be selected, or, in many cases, to be developed for the specific purpose. Thus, a diffusion and/or separation device for liquids usually only comprises a single type of hollow fiber membranes.

EP 1 201 293 A1 discloses a bundle of hollow fiber membranes intended to constitute the membrane of a device for treating blood or plasma by extracorporeal circulation in which the hydraulic permeability of the hollow fibers in the bundle is heterogeneous and the ratio of the highest hydraulic permeability measured on some hollow fibers of the bundle to the lowest hydraulic permeability measured on other hollow fibers in the same bundle is at least 5. The bundle is obtained by preparing a bundle of hollow fibers with a heterogeneous distribution of fibers within the bundle insofar as the density of the hollow fibers is higher in certain zones of the bundle than in other zones; mounting the bundle of hollow fibers in a tubular casing comprising two axial openings; causing a hot, dry gas that is chemically inert towards the hollow fibers to circulate through the bundle of hollow fibers not held at its ends, at a temperature and flow rate that are suitable to cause geometrical heterogeneity of the hollow fibers in the bundle as regards the internal diameter and wall thickness of the hollow fibers; stopping the hot dry gas from circulating when the geometrical heterogeneity of the hollow fibers has been obtained. The effect of the treatment is said to be that hydraulic permeability and transmittance of the membrane in the device can be adjusted independently of each other to a certain extent such that the hydraulic permeability of the membrane is low flux, medium flux or high flux, while the transmittances, in particular as regards toxins and proteins, are maintained at satisfactory values.

US 6 271 023 B1 discloses a membrane module which contains at last two groups of hollow fiber membranes arranged in layers and capable of being fed independently by fluids. The membrane module has a housing shell comprising a channel-shaped housing middle section, open at the top, and adjoining end pieces, also open at the top and with a number of arms corresponding to the number of groups. The groups of hollow fiber membranes are inserted as layers over one another such that they are arranged in the direction of the longitudinal extent of the housing middle section and substantially parallel to each other. The ends of hollow fiber membranes of different groups are arranged in respectively different arms of the end pieces and embedded with a sealing compound such that at least one end of the hollow fiber membranes is open.

US 2009/139925 A1 discloses a multi-zone polymer hollow fiber membrane and a dialyser comprising said membrane. The membrane is heat treated or portions of it are coated with polymer coatings, so that it has at least two zones with pores of different pore sizes.

US 6 214 232 B1 discloses an apparatus for substance-specific treatment of fluids, comprising a housing with a distribution space and a collection space, between which is arranged a structure of adjacent flat first and second layers containing first hollow fibers open in the direction of the distribution space and closed in the direction of the collection space, and second hollow fiber membranes open in the direction of the collection space and closed in the direction of the distribution space, respectively.

US 4 880 440 A discloses hollow fiber multi-component cells and permeator modules comprising two or more different permeable membranes capable of separating different components of a fluid mixture, in particular a mixture of gaseous compounds, for instance natural gas, producer gas, reactor gas etc. Separate fluid permeate streams are obtained from a single feed of a multi-component fluid mixture. The permeation cells are produced by winding two or more different hollow fibers on a shaft and building an annular bundle.

WO 91/04758 A1 discloses a combined blood heater/oxygenator device which has a generally cylindrical shape and a blood flow path which extends radially from a central blood distribution chamber through a first annular bundle of longitudinally extending heat exchange fibers and then through a second annular bundle of longitudinally extending gas exchange fibers.

It has now been found that separation properties of a diffusion and/or filtration device can be fitted to show the desired separation characteristics very efficiently, if at least two different types of hollow fiber membranes with differing separation properties are present in the hollow fiber membrane bundle.

## Summary

It is an object of the present invention to improve upon a generic filter device, so that it exhibits desired separation characteristics.

According to one aspect of the invention, a diffusion and/or filtration devices according to claim 1 is provided.

## Brief Description of the Drawings

Figure 1 shows sieving coefficients of albumin and ovalbumin in aqueous solution as well as liquid permeability of devices comprising fibers taken from Theralite® and Polyflux® P170H filters;

Figure 2 shows sieving coefficients of albumin and ovalbumin in aqueous solution as well as liquid permeability of devices comprising fibers taken from Theralite® and Xenium® 210 filters;

Figure 3 shows sieving coefficients of albumin and ovalbumin in aqueous solution as well as liquid permeability of devices comprising fibers taken from Theralite® and FX-800 filters;

Figure 4 shows sieving coefficients of albumin and ovalbumin in aqueous solution as well as liquid permeability of devices comprising fibers taken from Theralite® and Nephral® ST filters;

Figure 5 shows sieving coefficients of albumin and ovalbumin in aqueous solution as well as liquid permeability of devices comprising fibers taken from Theralite® and Polyflux® P21L filters;

Figure 6 shows sieving coefficients of albumin and ovalbumin in aqueous solution as well as liquid permeability of devices comprising fibers taken from Theralite® and Polyflux® HD-C4 filters;

Figure 7 shows a schematic view of a setup for producing a fiber strand comprising two different types of fibers. The fiber strand produced is comprised of 25% fibers of type A and 75% of fibers of type B.

## Detailed Description

One aspect of the present invention is a diffusion and/or filtration device comprising a tubular housing, a bundle of hollow fiber membranes arranged within the housing, and end caps sealing the mouths of the tubular housing, characterized in that the bundle of semi-permeable hollow fiber membranes comprises at least two different types of hollow fiber membranes uniformly distributed throughout the entire bundle. The diffusion and/or filtration device of the invention comprises:

a) housing means, said housing means defining a longitudinally extending internal chamber including a first end and a second end;

b) a bundle of semi-permeable hollow fiber membranes disposed within said internal chamber, said hollow fibers extending longitudinally from said first end of said housing to said second end of said housing, said hollow fiber membranes having an outer surface, and a first end and a second end corresponding to said first end and said second end of said internal chamber, the lumen of each of the hollow fiber membranes being in fluid connection to both the first and the second end of the internal chamber;

c) end wall means supporting said first and second ends of said hollow fiber membranes within said internal chamber so as to sealingly separate said first and second ends of said hollow fiber membranes from said outer surface of said hollow fiber between said first and second ends thereof;

d) first inlet means for the introduction of a fluid into said first end of said housing means, said first inlet means being defined by a first end cap covering said first end of said housing;

e) first outlet means for the evacuation of a fluid from said second end of said housing means, said first outlet means being defined by a second end cap covering said second end of said housing, said first and second end caps being applied to said first and second ends of said housing in a fluid-tight manner;

f) second outlet means for the evacuation of a fluid from said internal chamber at a location between said first and second end of said housing means.

In one embodiment, the diameter of the housing is not uniform. The housing has a middle section where the diameter is smaller than at the ends of the housing. Accordingly, the distances between the individual hollow fibers are smaller in the middle section of the device than at the end faces of the hollow fiber bundle In another embodiment, the housing has a diameter-expanding portion allowing hollow fiber membranes to be placed in a way that the distances between the hollow fiber membranes are gradually increased toward the end faces of the hollow fiber bundle.

The housing and end caps of the device of the invention are usually made of a transparent polymer, e.g. polyethylene, polypropylene, polyesters like PET or PBT, polymethyl-(meth)acrylate, polystyrene (HIPS) or polycarbonate. The potting material for the hollow fiber membranes usually is polyurethane. In one embodiment of the device of the invention, the housing and the end caps are made of polycarbonate, the potting material forming the end wall means is made of polyurethane.

A characteristic feature of the device of the invention is that the bundle of hollow fiber membranes comprises at least two different types of hollow fiber membranes. The individual types of hollow fiber membranes are distinguishably different from each other with regard to at least one property.

Examples of properties distinguishing two types of hollow fiber membranes are chemical composition, structure or texture, surface properties, fiber geometry or performance properties.

For instance, the individual types of membranes may be comprised of different polymers; or they may comprise different polymers; or they may comprise the same polymers but in different proportions.

Examples of suitable polymers that may be present in the membranes comprise polysulfone (PSU), polyethersulfone (PES), polyarylethersulfone (PAES); polyvinylpyrrolidone (PVP); polyurethane (PUR); polyamide (PA); polymethylmethacrylate (PMMA); polycarbonate (PC); polyacrylonitrile (PAN); polyester polymer alloy (PEPA), polyvinyl acetate (PVA); cellulose or cellulose derivatives such as cellulose acetate (CA), cellulose nitrate or regenerated cellulose; polyolefins like polyethylene (PE), polypropylene (PP), or polystyrene (PS); and fluoropolymers, such as polytetrafluoroethylene (PTFE) or polyvinylidene fluoride (PVDF). For instance, a bundle of hollow fibers may comprise one type of membranes comprising PAES and PVP and another type of membranes comprising PAN.

The individual types of membranes may have different structures or textures. The structure of the membranes may be symmetric or asymmetric; they may have a layered structure comprising different layers or a different number of layers; the membrane structure may comprise finger-like structures or sponge-like structures. For instance, a bundle of hollow fibers may comprise one type of membranes having an asymmetric structure and comprising a layer comprising finger-like structures and another type of membranes having a symmetric structure and comprising a layer of a sponge-like structure.

The individual types of membranes also may have different surface properties. For instance, the surface may be uniform or comprised of a plurality of individual domains; it may be uncharged or carry localized charges, either positive or negative charges; it may be uncoated or coated; it may have hydrophilic or hydrophobic properties; or it may carry functional groups, e.g. groups which are capable of binding to particular binding partners like toxins or antibodies. For instance, a bundle of hollow fibers may comprise one type of membranes having a hydrophilic surface and another type of membranes having a hydrophobic surface. In another embodiment, a bundle of hollow fibers may comprise one type of membranes having an uncharged surface and another type of membranes having a surface bearing localized charges.

The individual types of membranes also may have different fiber geometry, i.e. different internal diameter (ID), external diameter (OD), wall thickness, cross sectional shape, pore diameters or distribution of pore diameters etc. For instance, a bundle of hollow fibers may comprise one type of membranes having pores in the nanometer range and another type of membranes having pores in the micrometer range. As fiber geometry is reflected in the performance properties of the membranes, these properties can be used to distinguish between different types of membranes.

One such property is the hydraulic permeability Lp of the membranes. In the context of the present invention, two types of membranes are considered to be distinct if their hydraulic permeabilities, measured on mini-modules containing the individual membranes, as described in detail below, differ by at least 20% and at least $5*10^{-4}$ cm/bar*s.

In one embodiment, the bundle of hollow fibers in the device comprises at least one type of membranes having a hydraulic permeability of at least $50*10^{-4}$ cm/bar*s and at least one other type of membranes having a hydraulic permeability of less than $40*10^{-4}$ cm/bar*s.

Sieving coefficients may also be used to distinguish between different types of membranes. In the context of the present invention, two types of membranes are considered to be distinct if at least one of their sieving coefficients for myoglobin, ovalbumin, and albumin, respectively, measured on mini-modules containing the individual membranes, as described below, differ by at least 20%.

The proportion of the different types of hollow fibers in the bundle of hollow fibers in the device can vary over a certain range. In one embodiment of the invention, the percentages of the different types of hollow fibers present in the bundle are equal. In another embodiment, the majority of the fibers in the bundle, for instance 70%, 80% or even 90% or more, belongs to one type of fibers, and the other types of fibers present in the bundle together make up the rest. In an exemplary embodiment having a bundle of hollow fibers comprising two different types of fibers, one type may be present in a proportion of 70% or more, for instance 80% or more, or 90% or more, or even 95% of the total number of fibers, whereas the second type of fibers is present in a proportion of 30% or less, for instance 20% or less, or 10% or less, or even 5% of the total number of fibers.

The different types of fibers in the bundle of hollow fibers of the present invention are uniformly distributed throughout the entire bundle. In the context of the present invention, "uniformly distributed" means that the proportion of each individual type of fibers is more or less constant throughout the entire bundle. In other words, when looking at a cross-section of the bundle of hollow fibers, the ratio of the individual types of fibers present in any given circular subsection of said cross-section having a radius of 10 mm does not differ by more than 20%, for instance, not more than 10%, from the ratio in other similar subsections of said cross-section.

One example of a type of fibers that can be used in the device of the present invention is a membrane comprising polyethersulfone, polyamide, and polyvinylpyrrolidone having an asymmetric 3-layer structure and showing a hydraulic permeability Lp of about $5*10^{-4}$ cm/bar*s, available from Gambro and e.g. contained in filters sold under the trade name Polyflux® P21L.

Another example of a type of fibers that can be used in the device of the present invention is a membrane comprising polyethersulfone, polyamide, and polyvinylpyrrolidone having an asymmetric 3-layer structure and showing a hydraulic permeability Lp of about $80*10^{-4}$ cm/bar*s, available from Gambro and e.g. contained in filters sold under the trade name Polyflux® P210H.

Another example of a type of fibers that can be used in the device of the present invention is a membrane comprising polyarylethersulfone and polyvinylpyrrolidone and having an asymmetric 3-layer structure and showing a hydraulic permeability Lp of about $80*10^{-4}$ cm/bar*s, available from Gambro and e.g. contained in filters sold under the trade name Polyflux® Revaclear.

Another example of a type of fibers that can be used in the device of the present invention is a membrane comprising polyarylethersulfone and polyvinylpyrrolidone and having an asymmetric 3-layer structure and showing a hydraulic permeability Lp of about $480*10^{-4}$ cm/bar*s available from Gambro and e.g. contained in filters sold under the trade name Theralite®.

Another example of a type of fibers that can be used in the device of the present invention is a membrane made from a copolymer of acrylonitrile and sodium methallyl sulfonate, also known as AN69 membrane, having a homogeneous gel-structure, available from Gambro and e.g. contained in filters sold under the trade name Filtral®.

Another example of a type of fibers that can be used in the device of the present invention is a membrane made from a copolymer of acrylonitrile and sodium methallyl sulfonate which has a homogeneous gel-structure and is coated with polyethyleneimine, also known as AN69ST membrane, available from Gambro and e.g. contained in filters sold under the trade name Nephral® ST.

Another example of a type of fibers that can be used in the device of the present invention is a membrane comprising polysulfone and polyvinylpyrrolidone and having a nearly uniform sponge-like structure, available from Fresenius Medical Care under the trade name Helixon®.

Another example of a type of fibers that can be used in the device of the present invention is a membrane comprising polyethersulfone and polyvinylpyrrolidone and having a nearly uniform sponge-like structure, available from Membrana GmbH, 42289 Wuppertal, Germany, under the trade name PUREMA®.

The device of the present invention can be produced using methods known in the art. The person skilled in the art will not have difficulties in selecting the appropriate equipment and production methods in accordance with the nature and type of device to be produced. The manufacturing process for the device according to the present invention uses different types of fibers provided on individual spools. The spools are put on a rack and the fibers from the individual spools are simultaneously pulled onto a winding wheel, producing a fiber bundle among which the individual types of fibers are homogeneously distributed. The winding wheel is rotated until a fiber strand comprising the desired number of fibers has been obtained.

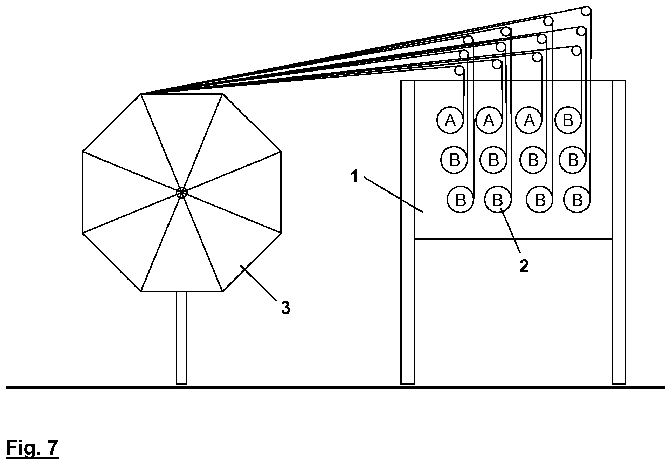

A schematic view of an example of the setup is shown in Fig. 7. The spool rack (1) carries 12 spools (2) in total, 3 spools with fibers of type A and 9 spools of fibers of type B, and every spool (2) delivers one fiber. The final fiber bundle on the winding wheel (**3**) will contain 25% fibers of type A and 75% fibers of type B. After 700 turns of the winding wheel (**3**), a fiber strand with 8,400 fibers is created, comprising fibers of type A and fibers of type B, homogeneously distributed across the fiber strand.

The fiber strand is subsequently cut into bundles of appropriate length, e.g. 320 mm, and each bundle is transferred into a housing, for instance, a cylindrical housing. To facilitate transfer from the winding wheel, the fiber strand may be wrapped in a polymer film before cutting.

Both ends of the fiber bundle in the housing are potted with a reactive polymer system, e.g., polyurethane resin or epoxy resin, to produce two separated fluid compartments, one connected to the lumen surface of the hollow fibers and the other connected to the outside surface of the hollow fibers. After the reactive polymer system is cured, the ends of the fiber bundle are cut to re-open the fibers.

Subsequently, assembly of the device is completed as known in the art, e.g., by mounting end caps on the housing, adding connectors, etc.

One embodiment of the invention is a device comprising (a) hollow fiber membranes capable of removing cell-activating molecules such as cytokines, for instance fibers having a sieving coefficient for ovalbumin in aqueous solution of 0.80 or more and a sieving coefficient for albumin in aqueous solution of 0.40 or less (e.g., fibers contained in filters sold under the trade name Theralite®), and (b) hollow fibers carrying functional groups and/or ligands capable of binding to endotoxins. The device optionally comprises (c) means for removing activated leukocytes from blood, for instance a non-woven melt-blown web of cycloolefin copolymer fibers coated with Tween® 20 (as described in WO 2007/25738 A2) disposed in at least one of the end caps of the device.

The device is useful for treating patients suffering from chronic or acute inflammatory diseases, e.g., sepsis, acute kidney injury (AKI), rheumatoid arthritis, or inflammatory bowel diseases like Crohn's disease or ulcerative colitis. It is envisioned that the device provides for an improved therapy of inflammatory diseases by extracorporeal blood treatment, as it is able to simultaneously remove both endotoxins and cell-activating molecules such as cytokines, and, if (c) means for removing activated leukocytes from blood are present in the device, even activated cells. Of course, (c) means for removing activated leukocytes can also be implemented in devices which only comprise either fibers of type (a) or type (b), respectively, or devices which comprise particles carrying functional groups and/or ligands capable of binding to endotoxins, for instance, columns packed with functionalized polymer beads. Also in these cases, an additional benefit to the patient is expected, as the removal of activated leukocytes in addition to the removal of cell-activating molecules or endotoxins, respectively, will help to normalize the inflammatory status of the patient more quickly.

It will be understood that the features mentioned above and those described hereinafter can be used not only in the combination specified but also in other combinations or on their own, without departing from the scope of the present invention.

The present invention will now be described in more detail in the examples below. It is to be understood that the examples are not intended to limit the scope of the present invention and are merely an illustration of a preferred embodiment of the invention.

**Examples**

**Preparation of mini-modules:**

Mini-modules were prepared from commercially available filters. The number of fibers required for a mini-module having a surface A of 360 cm$^2$ is calculated according to equation 1:

$$A = \pi \times d_i \times l \times n \tag{1}$$

with

$d_i$ = inner diameter of fiber [cm]
$n$ = number of fibers
$l$ = fiber length [cm]

In case of Nephral® ST (AN69ST) filters, the blood compartment of the filter was rinsed by recirculation with one liter of aqueous glycerol solution (60% w/w) at 50 to 100 ml/min whilst the dialysate compartment was closed to avoid ultrafiltration. After one hour, the blood compartment was purged with cold air at a pressure of approx. one bar for 20 minutes. All other filters were used as received.

The housings of the filters were cut open and the fibers were cut out. For each mini-module comprising two different types of fibers, the required number of fibers from the respective filters was collected and both fractions were mixed to obtain a homogeneous bundle of fibers. The fiber bundle was cut to a defined length of 20 cm and transferred into a mini-module housing. The fiber ends were sealed by melting and the ends of the fiber bundle were transferred into a potting cap. In case of mini-modules comprising AN69ST membranes, the bundles were purged with cold air for 20 minutes before the potting process was started. The fibers were potted with polyurethane. After the polyurethane had hardened, the potted membrane bundle was cut to a defined length to open the ends of the membrane fibers and stored dry before it was used for the performance tests. The minimal distance between the polyurethane surfaces of the potted ends was taken as the effective fiber length for calculating the effective surface of the mini-module using equation 1.

## Measurement of hydraulic permeability (Lp)

The mini-module is immersed in RO water for 30 minutes. Then the module is mounted into the Lp test device in horizontal position under water thermostatted at 37 °C. The mini-module is checked for leakages by perfusion of the blood compartment with air, and then refilled with RO water. Then a defined amount of water (e.g. 1 $cm^3$) is filtered through the membrane at a suitable gage pressure (e.g. 130 mbar). The time required is measured and the hydraulic permeability Lp is calculated according to equation 2:

$$Lp = \frac{V}{p \cdot A \cdot t} = \frac{V}{\pi \cdot d \cdot l \cdot n \cdot p \cdot t} \tag{2}$$

with:

Lp =    hydraulic permeability [$*10^{-4}$ cm/(bar*s)]
V =    volume of water filtered through the membrane [$cm^3$]
p =    applied pressure [bar]
t =    duration of measurement [s]
A =    effective surface [$cm^2$]
d =    inner diameter [cm]
l =    effective fiber length [cm]
n =    number of fibers

## Measurement of sieving coefficients in aqueous solution

Sieving coefficients $SC_{ov}$ for ovalbumin (45kDa) and $SC_{alb}$ for albumin (66kDa) are measured on the same mini-module, measurement of $SC_{ov}$ preceding measurement of $SC_{alb}$. The mini-module is perfused with PBS buffer (approx. 30 ml) before each test. All test solutions are thermostatted at approx. 37 °C. The test solutions are pumped through the blood compartment of the mini-module by means of two variable pumps located upstream and downstream, respectively, of the mini-module. After 15 minutes, samples of the feed solution entering the inlet of the module, the filtrate and the retentate leaving the outlet of the module are taken, the protein concentrations in the respective solutions are determined and the sieving coefficient is calculated according to equation 3:

$$sc = \frac{2 \cdot C_F}{C_{in} + C_{out}} \tag{3}$$

with

sc =    sieving coefficient [%]
$C_F$ =    filtrate concentration
$C_{in}$ =    concentration in inlet solution
$C_{out}$ =    concentration in outlet solution

Suitable PBS buffer can, for instance, be prepared from concentrate (e.g. Lonza BE17-517Q) by dilution with RO water. Albumin concentrations can, for instance, be determined by photometry using the biuret method; and ovalbumin concentrations can, for instance, be determined by photometry using the Bradford test (e.g., with the Bio-Rad protein assay).

**Measurement of $sc_{ov}$**

The test solution (100 ml/l ovalbumin in PBS buffer) is perfused single pass through the blood compartment of the mini-module at a fixed shear rate of 500 s$^{-1}$ and a fixed intrinsic flow rate of 0.38 *10$^{-4}$ cm/s. The inlet flow $Q_B$ required for achieving a shear rate of 500 s$^{-1}$ is calculated according to equation 4:

$$Q_B = \frac{\gamma \cdot n \cdot \pi \cdot d_i^3 \cdot 60 \, s \, / \, min}{32} \qquad (4)$$

with

$Q_B$ = inlet flow [ml/min]
$\gamma$ = shear rate [s$^{-1}$]
n = number of fibers
$d_i$ = inner diameter [cm]

The ultrafiltration rate required for achieving an intrinsic flow rate of 0.38 *10$^{-4}$ cm/s is calculated according to equation 5:

$$Q_{UF} = J_v \cdot A \cdot 60 \, s \, / \, min \qquad (5)$$

with

$Q_{UF}$ = ultrafiltration rate [ml/min]
$J_v$ = intrinsic flow rate = [cm/s]
A = effective surface [cm$^2$]

**Measurement of $sc_{alb}$**

The test solution (60 g/l albumin in PBS buffer) is perfused through the mini-module, and filtrate and retentate are recirculated to the reservoir of the test solution. Fluid pressure is measured at the inlet and the outlet of the mini-module, and the trans-membrane pressure (TMP = ($p_{in}$ + $p_{out}$)/2) is set to 400 mmHg (0.53 bar) gage pressure.

**Example 1**

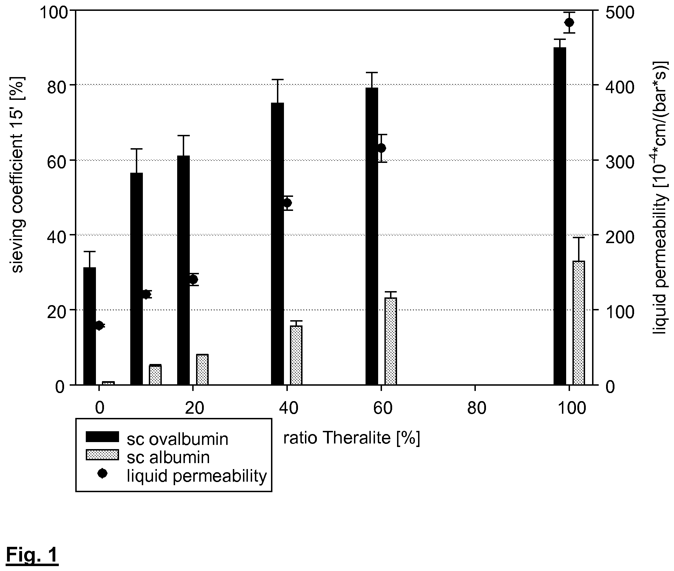

Hollow fiber membranes were extracted from two different commercially available dialysers, a Theralite® dialyser and a Polyflux® 170H dialyser, both available from Gambro. Mini-modules were prepared as described above using different proportions of the two fiber types ranging from 0/100 to 100/0 percent. Lp [10$^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 1 and Fig. 1. The values represent the mean values of three determinations. As is evident from the data, when the proportion of Theralite® fibers in the device is increased, liquid permeability and sieving coefficient of albumin grow linearly with the percentage of Theralite® fibers, but a disproportionately high increase in the sieving coefficient of ovalbumin is observed. Thus, removal of ovalbumin is drastically improved even at low percentages of Theralite® fibers, while loss of albumin only increases slightly.

Tab. 1 Properties of mini-modules comprising fibers from Theralite® and Polyflux® 170H dialysers

| Theralite-P170H | Lp | std. dev. | $sc_{ov}$ | std. dev. | $sc_{alb}$ | std. dev. |
|---|---|---|---|---|---|---|
| 0%-100% | 79.2 | 1.6 | 31.2 | 4.3 | 0.7 | 0.1 |
| 10%-90% | 120.6 | 4.7 | 56.4 | 4.3 | 5.0 | 0.4 |
| 20%-80% | 140.6 | 9.1 | 60.9 | 6.5 | 8.0 | 0.1 |
| 40%-60% | 242.5 | 18.3 | 75.1 | 5.6 | 15.7 | 1.4 |
| 60%-40% | 315.6 | 3.8 | 79.1 | 6.3 | 23.1 | 1.7 |

(continued)

| Theralite-P170H | Lp | std. dev. | $sc_{ov}$ | std. dev. | $sc_{alb}$ | std. dev. |
|---|---|---|---|---|---|---|
| 100%-0% | 483.3 | 13.8 | 89.8 | 2.4 | 33.0 | 6.3 |

## Example 2

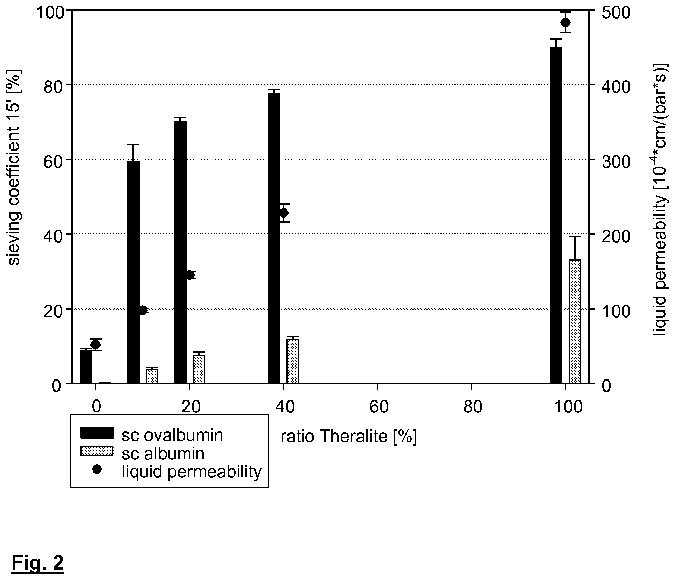

Hollow fiber membranes were extracted from two different commercially available dialysers, a Theralite® dialyser, available from Gambro, and a Xenium® 210G dialyser, which comprised polyethersulfone hollow fiber membranes having an inner diameter of 198 $\mu$m and a wall thickness of 34 $\mu$m, available from Baxter Deutschland GmbH. Mini-modules were prepared as described above using different proportions of the two fiber types ranging from 0/100 to 100/0 percent. Lp [$10^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 2 and Fig. 2. The values represent the mean values of three determinations. As is evident from the data, when the proportion of Theralite® fibers in the device is increased, liquid permeability and sieving coefficient of albumin grow linearly with the percentage of Theralite® fibers, but a disproportionately high increase in the sieving coefficient of ovalbumin is observed. Thus, removal of ovalbumin is drastically improved even at low percentages of Theralite® fibers, while loss of albumin only increases slightly.

Tab. 2 Properties of mini-modules comprising fibers from Theralite® and Xenium® 210G dialysers

| Theralite-Xenium | Lp | std. dev. | $sc_{ov}$ | std. dev. | $sc_{alb}$ | std. dev. |
|---|---|---|---|---|---|---|
| 0%-100% | 52.0 | 7.6 | 8.9** | 0.4** | 0.3 | 0.0 |
| 10%-90% | 98.0 | 2.7 | 59.3 | 4.7 | 3.9 | 0.4 |
| 20%-80% | 145.2 | 4.6 | 70.1 | 1.0 | 7.4 | 1.0 |
| 40%-60%* | 228.3 | 12.0 | 77.5 | 1.3 | 11.8 | 0.8 |
| 100%-0% | 483.3 | 13.8 | 89.8 | 2.4 | 33.0 | 6.3 |
| * only two determinations | | | | | | |
| ** extrapolated values, as the concentration was below the determination limit of 20mg/L | | | | | | |

## Example 3

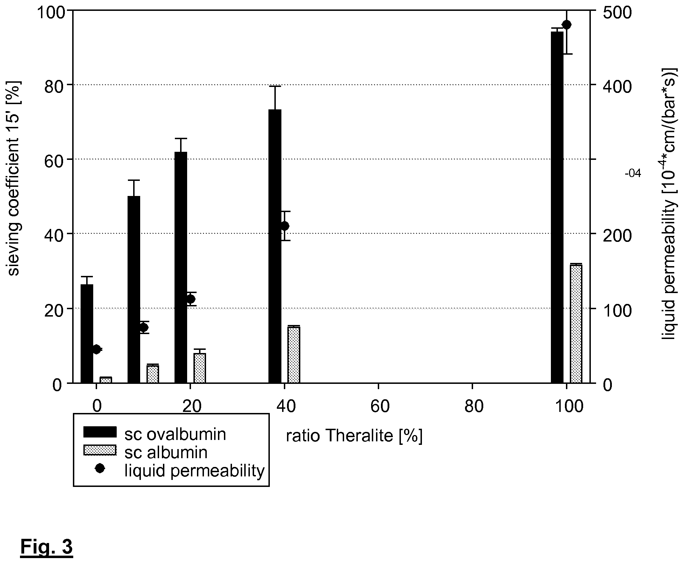

Hollow fiber membranes were extracted from two different commercially available dialysers, a Theralite® dialyser, available from Gambro, and a FX-800 dialyser, which comprised polysulfone hollow fiber membranes having an inner diameter of 198 $\mu$m and a wall thickness of 43 $\mu$m, available from Fresenius Medical Care. Mini-modules were prepared as described above using different proportions of the two fiber types ranging from 0/100 to 100/0 percent. Lp [$10^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 3 and Fig. 3. The values represent the mean values of three determinations As is evident from the data, when the proportion of Theralite® fibers in the device is increased, liquid permeability and sieving coefficient of albumin grow linearly with the percentage of Theralite® fibers, but a disproportionately high increase in the sieving coefficient of ovalbumin is observed. Thus, removal of ovalbumin is drastically improved even at low percentages of Theralite® fibers, while loss of albumin only increases slightly..

Tab. 3 Properties of mini-modules comprising fibers from Theralite® and FX-800 dialysers

| Theralite-FX-800 | Lp | std. dev. | $sc_{ov}$ | std. dev. | $sc_{alb}$ | std. dev. |
|---|---|---|---|---|---|---|
| 0%-100% | 45.0 | 1.2 | 26.3 | 2.2 | 1.3 | 0.2 |
| 10%-90% | 74.5 | 8.0 | 49.9 | 4.4 | 4.6 | 0.4 |
| 20%-80% | 112.6 | 8.8 | 61.7 | 3.8 | 7.9 | 1.2 |
| 40%-60%* | 210.1 | 19.4 | 73.1 | 6.4 | 14.9 | 0.5 |

(continued)

| Theralite-FX-800 | Lp | std. dev. | $sc_{ov}$ | std. dev. | $sc_{alb}$ | std. dev. |
|---|---|---|---|---|---|---|
| 100%-0%* | 480.3 | 39.2 | 94.0 | 1.1 | 31.5 | 0.4 |
| * only two determinations | | | | | | |

### Example 4

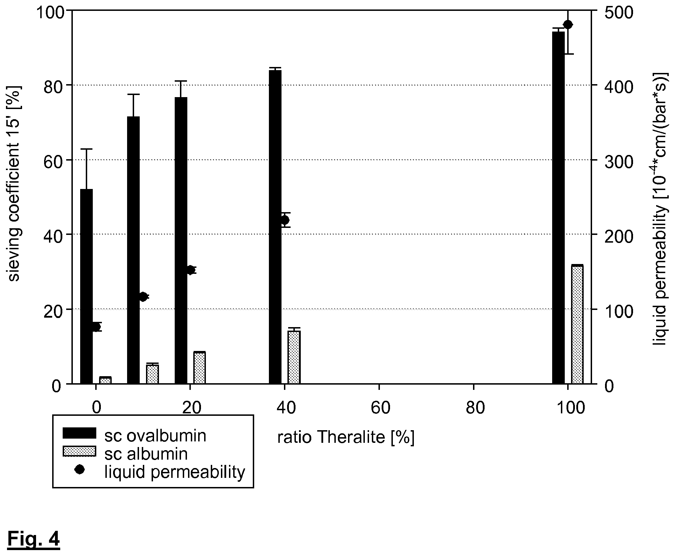

Hollow fiber membranes were extracted from two different commercially available dialysers, a Theralite[®] dialyser and a Polyflux[®] HD-C4 dialyser, both available from Gambro. Mini-modules were prepared as described above using different proportions of the two fiber types ranging from 0/100 to 100/0 percent. Lp [$10^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 4 and Fig. 4. The values represent the mean values of three determinations. As is evident from the data, when the proportion of Theralite[®] fibers in the device is increased, liquid permeability and sieving coefficient of albumin grow linearly with the percentage of Theralite[®] fibers, but a disproportionately high increase in the sieving coefficient of ovalbumin is observed. Thus, removal of ovalbumin is drastically improved even at low percentages of Theralite[®] fibers, while loss of albumin only increases slightly.

Tab. 4 Properties of mini-modules comprising fibers from Theralite[®] and Polyflux[®] HD-C4 dialysers

| Theralite-HD-C4 | Lp | std. dev. | $sc_{ov}$ | std. dev. | $sc_{alb}$ | std. dev. |
|---|---|---|---|---|---|---|
| 0%-100% | 76.7 | 5.6 | 52.0 | 10.8 | 1.6 | 0.3 |
| 10%-90% | 116.7 | 2.0 | 71.3 | 6.2 | 5.0 | 0.6 |
| 20%-80% | 152.5 | 4.1 | 76.5 | 4.5 | 8.4 | 0.3 |
| 40%-60% | 219.0 | 9.7 | 83.7 | 0.8 | 14.1 | 1.0 |
| 100%-0%* | 480.3 | 39.2 | 94.0 | 1.1 | 31.5 | 0.4 |
| * only two determinations | | | | | | |

### Example 5

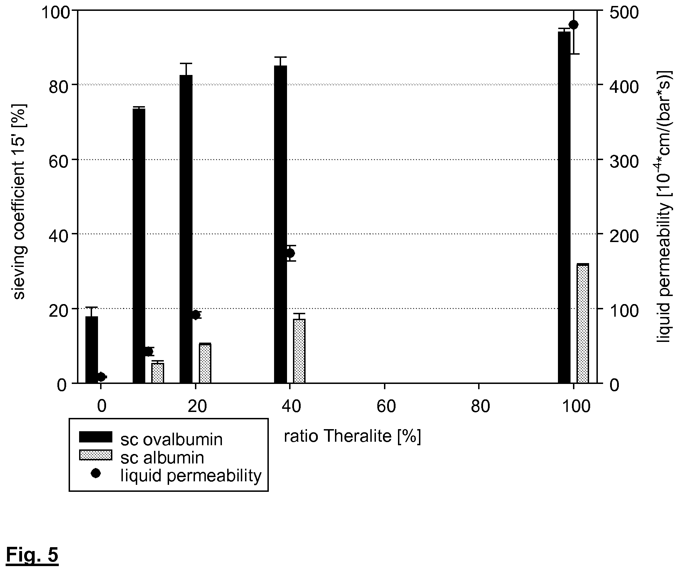

Hollow fiber membranes were extracted from two different commercially available dialysers, a Theralite[®] dialyser, and a Nephral[®] ST (AN69ST) dialyser, both available from Gambro. Mini-modules were prepared as described above using different proportions of the two fiber types ranging from 0/100 to 100/0 percent. Lp [$10^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 5 and Fig. 5. The values represent the mean values of three determinations. As is evident from the data, when the proportion of Theralite[®] fibers in the device is increased, liquid permeability and sieving coefficient of albumin grow linearly with the percentage of Theralite[®] fibers, but a disproportionately high increase in the sieving coefficient of ovalbumin is observed. Thus, removal of ovalbumin is drastically improved even at low percentages of Theralite[®] fibers, while loss of albumin only increases slightly.

Tab. 5 Properties of mini-modules comprising fibers from Theralite[®] and Nephral[®] ST (AN69ST) dialysers

| Theralite-AN69ST | Lp | std. dev. | $sc_{ov}$ | std. dev. | $sc_{alb}$ | std. dev. |
|---|---|---|---|---|---|---|
| 0%-100% | 8.2 | 0.9 | 17.7** | 2.7** | 0.1 | 0.0 |
| 10%-90% | 42.2 | 5.4 | 73.3 | 0.7 | 5.2 | 0.8 |
| 20%-80% | 91.5 | 4.2 | 82.4 | 3.3 | 10.3 | 0.4 |
| 40%-60% | 174.0 | 10.4 | 84.9 | 2.4 | 17.1 | 1.6 |
| 100%-0%* | 480.3 | 39.2 | 94.0 | 1.1 | 31.5 | 0.4 |
| * only two determinations<br>** extrapolated values, as the concentration was below the determination limit of 20mg/L | | | | | | |

## Example 6

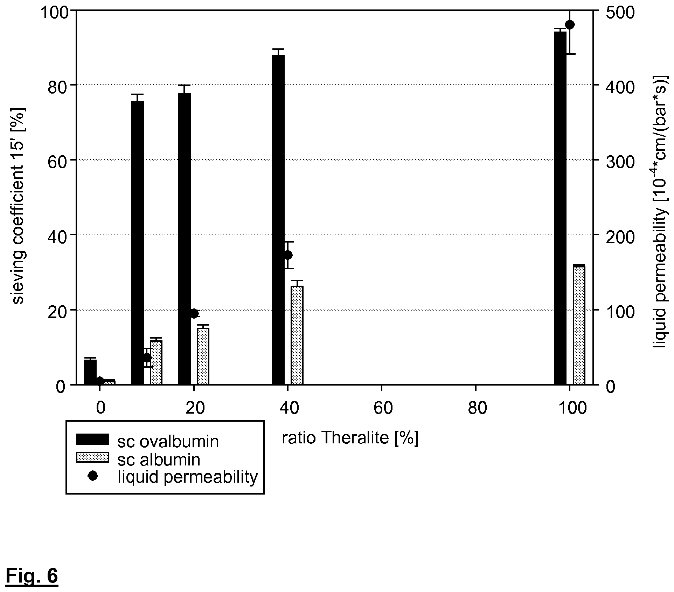

Hollow fiber membranes were extracted from two different commercially available dialysers, a Theralite® dialyser and a Polyflux® 21L dialyser, both available from Gambro. Mini-modules were prepared as described above using different proportions of the two fiber types ranging from 0/100 to 100/0 percent. Lp [$10^{-4}$*cm/(bar*s)] and sieving coefficients for albumin ($sc_{alb}$ [%]) and ovalbumin ($sc_{ov}$ [%]) in aqueous solution after 15 minutes were determined for the mini-modules as described above. The results are shown in Table 6 and Fig. 6. The values represent the mean values of three determinations. As is evident from the data, when the proportion of Theralite® fibers in the device is increased, liquid permeability and sieving coefficient of albumin grow linearly with the percentage of Theralite® fibers, but a disproportionately high increase in the sieving coefficient of ovalbumin is observed. Thus, removal of ovalbumin is drastically improved even at low percentages of Theralite® fibers, while loss of albumin only increases slightly.

Tab. 6 Properties of mini-modules comprising fibers from Theralite® and Polyflux® 21L dialysers

| Theralite-P21L | Lp | std. dev. | $sc_{ov}$ | std. dev. | $sc_{alb}$ | std. dev. |
|---|---|---|---|---|---|---|
| 0%-100% | 4.5 | 0.2 | 6.5** | 0.7** | 0.9 | 0.4 |
| 10%-90% | 35.9 | 12.2 | 75.4 | 2.1 | 11.6 | 0.9 |
| 20%-80% | 95.0 | 3.9 | 77.6 | 2.3 | 15.0 | 1.0 |
| 40%-60% | 172.9 | 17.8 | 87.7 | 1.8 | 26.2 | 1.6 |
| 100%-0%* | 480.3 | 39.2 | 94.0 | 1.1 | 31.5 | 0.4 |

\* only two determinations
\*\* extrapolated values, as the concentration was below the determination limit of 20mg/L

## Claims

1. A diffusion and/or filtration device comprising:

   a) housing means, the housing means defining a longitudinally extending internal chamber including a first end and a second end;
   b) a bundle of semi-permeable hollow fiber membranes disposed within the internal chamber, the hollow fiber membranes extending longitudinally from the first end of the housing to the second end of the housing, the hollow fiber membranes having an outer surface, and a first end and a second end corresponding to the first end and the second end of the internal chamber, the lumen of each of the hollow fiber membranes being in fluid connection to both the first end and the second end of the internal chamber;
   c) end wall means supporting the first and second ends of the hollow fiber membranes within the internal chamber so as to sealingly separate the first and second ends of the hollow fiber membranes from the outer surface of the hollow fiber membranes between the first and second ends thereof;
   d) first inlet means for the introduction of a fluid into the first end of the housing means, the first inlet means being defined by a first end cap covering the first end of the housing;
   e) first outlet means for the evacuation of a fluid from the second end of the housing means, the first outlet means being defined by a second end cap covering the second end of the housing, the first and second end caps being applied to the first and second ends of the housing in a fluid-tight manner;
   f) second outlet means for the evacuation of a fluid from the internal chamber at a location between the first and second end of the housing means;

   **characterized in that** the bundle of semi-permeable hollow fiber membranes comprises at least two different types of hollow fiber membranes uniformly distributed throughout the entire bundle.

2. The device of claim 1, wherein at least two types of hollow fibers differ from each other in chemical composition.

3. The device of claim 1 or 2, wherein at least two types of hollow fibers differ from each other in structure or texture.

4. The device of any one of claims 1 to 3, wherein at least two types of hollow fibers differ from each other in surface properties.

**5.** The device of any one of claims 1 to 4, wherein at least two types of hollow fibers differ from each other in fiber geometry.

**6.** The device of any one of claims 1 to 5, wherein at least two types of hollow fibers differ from each other in at least one of their performance properties.

**7.** The device of claim 6, wherein the performance property is hydraulic permeability Lp.

**8.** The device of claim 6, wherein the performance property is the sieving coefficient for albumin in aqueous solution.

**9.** The device of claim 6, wherein the performance property is the sieving coefficient for ovalbumin in aqueous solution.

**10.** The device of any one of claims 1 to 9, wherein the percentages of the different types of fibers present in the bundle of semi-permeable hollow fiber membranes are equal.

**11.** The device of any one of claims 1 to 9, wherein one type of fibers present in the bundle of semi-permeable hollow fiber membranes accounts for at least 70% of the total number of fibers, and the other types of fibers taken together make up the rest.

**12.** A process for preparing the device of any one of claims 1 to 11, comprising the steps of

a) providing a stock (**1,2**) of hollow fiber membranes of at least two different types of hollow fiber membranes;
b) withdrawing hollow fiber membranes from the stock(**1,2**) of hollow fiber membranes, with the proviso that the hollow fiber membranes withdrawn from the stock comprise at least two different types of hollow fiber membranes;
c) bundling the hollow fiber membranes withdrawn from the stock (**1,2**) using bundling means (**3**) to produce a strand of hollow fibers.

**13.** The process of claim 12, wherein the stock (**1,2**) of hollow fiber membranes comprises hollow fiber membranes wound on spools (**2**), the spools (**2**) being mounted on a rack (**1**) and the bundling means (**3**) comprise a winding wheel.

**14.** Use of a device according to any one of claims 1 to 11 in hemodialysis, hemodiafiltration, or hemofiltration.

**Patentansprüche**

**1.** Diffusions- und/oder Filtrationsvorrichtung umfassend:

a) Gehäusemittel, die eine sich in Längsrichtung erstreckende interne Kammer definieren, die ein erstes und ein zweites Ende aufweist;
b) ein in der internen Kammer angeordnetes Bündel semipermeabler Hohlfasermembranen, wobei die Hohlfasermembranen sich vom ersten Ende des Gehäuses zum zweiten Ende des Gehäuses erstrecken, die Hohlfasern eine äußere Oberfläche aufweisen und ein erstes und ein zweites Ende, die dem ersten und zweiten Ende der internen Kamer entsprechen, und das Lumen jeder der Hohlfasern in Fluidverbindung sowohl mit dem ersten wie auch dem zweiten Ende der internen Kammer steht;
c) Stirnwandmittel, welche die ersten und zweiten Enden der Hohlfasermembranen innerhalb der internen Kammer abstützen, so dass die ersten und zweiten Enden der Hohlfasermembranen von der äußeren Oberfläche der Hohlfasermembranen zwischen ihren ersten und zweiten Enden dicht abgeschottet sind;
d) erste Einlassmittel zur Einleitung eines Fluids in das erste Ende der Gehäusemittel, wobei die ersten Einlassmittel durch eine erste Endkappe definiert sind, die das erste Ende des Gehäuses abdeckt;
e) erste Auslassmittel zum Abtransport eines Fluids aus dem zweiten Ende der Gehäusemittel, wobei die ersten Auslassmittel durch eine zweite Endkappe definiert sind, die das zweite Ende des Gehäuses abdeckt, wobei die erste und die zweite Endkappe in fluiddichter Weise auf das erste und zweite Ende des Gehäuses aufgesetzt sind;
f) zweite Auslassmittel zum Abtransport eines Fluids aus der internen Kammer an einer Stelle zwischen dem ersten und zweiten Ende der Gehäusemittel;

**dadurch gekennzeichnet, dass** das Bündel semipermeabler Hohlfasermembranen mindestens zwei verschiedene Arten von Hohlfasermembranen umfasst, die gleichmäßig innerhalb des gesamten Bündels verteilt sind.

2. Vorrichtung gemäß Anspruch 1, worin mindestens zwei Arten von Hohlfasern sich durch ihre chemische Zusammensetzung unterscheiden.

3. Vorrichtung gemäß Anspruch 1 oder 2, worin mindestens zwei Arten von Hohlfasern sich durch ihre Struktur oder Textur unterscheiden.

4. Vorrichtung gemäß einem der Ansprüche 1 bis 3, worin mindestens zwei Arten von Hohlfasern sich durch ihre Oberflächeneigenschaften unterscheiden.

5. Vorrichtung gemäß einem der Ansprüche 1 bis 4, worin mindestens zwei Arten von Hohlfasern sich durch ihre Fasergeometrie unterscheiden.

6. Vorrichtung gemäß einem der Ansprüche 1 bis 5, worin mindestens zwei Arten von Hohlfasern sich in mindestens einer ihrer Leistungseigenschaften unterscheiden.

7. Vorrichtung gemäß Anspruch 6, worin die Leistungseigenschaft die hydraulische Permeabilität Lp ist.

8. Vorrichtung gemäß Anspruch 6, worin die Leistungseigenschaft der Siebkoeffizient für Albumin in wässriger Lösung ist.

9. Vorrichtung gemäß Anspruch 6, worin die Leistungseigenschaft der Siebkoeffizient für Ovalbumin in wässriger Lösung ist.

10. Vorrichtung gemäß einem der Ansprüche 1 bis 9, worin die Prozentanteile der unterschiedlichen Arten von in dem Bündel semipermeabler Hohlfasermembranen vorhandenen Fasern gleichgroß sind.

11. Vorrichtung gemäß einem der Ansprüche 1 bis 9, worin eine Art von in dem Bündel semipermeabler Hohlfasermembranen vorhandenen Fasern mindestens 70% der Gesamtzahl der Fasern ausmacht und die anderen Arten zusammengenommen den Rest ausmachen.

12. Verfahren zur Herstellung der Vorrichtung gemäß einem der Ansprüche 1 bis 11, umfassend die Schritte

a) Bereitstellen eines Vorrats (**1,2**) von Hohlfasermembranen mindestens zweier unterschiedlicher Arten von Hohlfasermembranen;
b) Entnehmen von Hohlfasermembranen aus dem Vorrat (**1,2**) von Hohlfasermembranen, mit der Maßgabe, dass die aus dem Vorrat entnommenen Hohlfasermembranen mindestens zwei unterschiedliche Arten von Hohlfasermembranen umfassen;
c) Bündeln der aus dem Vorrat (**1,2**) entnommenen Hohlfasermembranen unter Verwendung von Bündelmitteln (**3**), um einen Strang von Hohlfasern herzustellen.

13. Verfahren gemäß Anspruch 12, bei dem der Vorrat (**1,2**) von Hohlfasermembranen auf Spulen (**2**) aufgewickelte Hohlfasermembranen umfasst, die Spulen (**2**) auf einem Gestell (**1**) montiert sind und die Bündelmittel (**3**) ein Wickelrad umfassen.

14. Verwendung einer Vorrichtung gemäß einem der Ansprüche 1 bis 11 in der Hämodialyse, Hämodiafiltration oder Hämofiltration.


**Revendications**

1. Dispositif de diffusion et/ou de filtration, comprenant:

a) des moyens de boîtier, les moyens de boîtier définissant une chambre interne s'étendant longitudinalement, comportant une première extrémité et une seconde extrémité;
b) un faisceau de membranes de fibres creuses semi-perméables disposé à l'intérieur de la chambre interne, les membranes de fibres creuses s'étendant longitudinalement de la première extrémité du boîtier à la seconde extrémité du boîtier, les membranes de fibres creuses présentant une surface extérieure, et une première extrémité et une seconde extrémité correspondant à la première extrémité et à la seconde extrémité de la chambre interne, la lumière de chacune des membranes de fibres creuses étant en connexion fluidique à la fois avec la première extrémité et avec la seconde extrémité de la chambre interne;

c) des moyens de paroi d'extrémité supportant les première et seconde extrémités des membranes de fibres creuses à l'intérieur de la chambre interne de façon à séparer de façon étanche les première et seconde extrémités des membranes de fibres creuses de la surface extérieure des membranes de fibres creuses entre la première et la seconde extrémités de celles-ci;

d) des premiers moyens d'entrée pour l'introduction d'un fluide dans la première extrémité des moyens de boîtier, les premiers moyens d'entrée étant définis par une première coiffe d'extrémité couvrant la première extrémité du boîtier;

e) des premiers moyens de sortie pour l'évacuation d'un fluide de la seconde extrémité des moyens de boîtier, les premiers moyens de sortie étant définis par une seconde coiffe d'extrémité couvrant la seconde extrémité du boîtier, les première et seconde coiffes d'extrémité étant appliquées aux première et seconde extrémités du boîtier d'une manière étanche au fluide; et

f) des seconds moyens de sortie pour l'évacuation d'un fluide de la chambre interne à un endroit situé entre les première et seconde extrémités des moyens de boîtier,

**caractérisé en ce que** le faisceau de membranes de fibres creuses semi-perméables comprend au moins deux types différents de membranes de fibres creuses uniformément distribuées dans le faisceau tout entier.

2. Dispositif selon la revendication 1, dans lequel au moins deux types de fibres creuses diffèrent l'un de l'autre par leur composition chimique.

3. Dispositif selon la revendication 1 ou 2, dans lequel au moins deux types de fibres creuses diffèrent l'un de l'autre par leur structure ou leur texture.

4. Dispositif selon l'une quelconque des revendications 1 à 3, dans lequel au moins deux types de fibres creuses diffèrent l'un de l'autre par les propriétés de surface.

5. Dispositif selon l'une quelconque des revendications 1 à 4, dans lequel au moins deux types de fibres creuses diffèrent l'un de l'autre par la géométrie des fibres.

6. Dispositif selon l'une quelconque des revendications 1 à 5, dans lequel au moins deux types de fibres creuses diffèrent l'un de l'autre par au moins une de leurs propriétés de performance.

7. Dispositif selon la revendication 6, dans lequel la propriété de performance est la perméabilité hydraulique Lp.

8. Dispositif selon la revendication 6, dans lequel la propriété de performance est le coefficient de filtrage de l'albumine en solution aqueuse.

9. Dispositif selon la revendication 6, dans lequel la propriété de performance est le coefficient de filtration de l'ovalbumine en solution aqueuse.

10. Dispositif selon l'une quelconque des revendications 1 à 9, dans lequel les pourcentages des différents types de fibres présents dans le faisceau de membranes de fibres creuses semi-perméables sont égaux.

11. Dispositif selon l'une quelconque des revendications 1 à 9, dans lequel un type de fibres présent dans le faisceau de membranes de fibres creuses semi-perméables intervient pour au moins 70 % du nombre total de fibres, et les autres types de fibres pris ensemble constituent le reste.

12. Procédé pour préparer un dispositif selon l'une quelconque des revendications 1 à 11, comprenant les étapes suivantes:

a) fournir un stock (1, 2) de membranes de fibres creuses d'au moins deux types différents de membranes de fibres creuses;

b) retirer des membranes de fibres creuses hors du stock (1, 2) de membranes de fibres creuses, avec la condition que les membranes de fibres creuses retirées hors du stock comprennent au moins deux types différents de membranes de fibres creuses; et

c) regrouper les membranes de fibres creuses retirées du stock (1, 2) en utilisant des moyens de regroupement (3) pour produire une botte de fibres creuses.

**13.** Procédé selon la revendication 12, dans lequel le stock (1, 2) de membranes de fibres creuses comprend des membranes de fibres creuses enroulées sur des bobines (2), les bobines (2) étant montées sur une crémaillère (1) et les moyens de regroupement (3) comprenant une roue d'enroulement.

**14.** Utilisation d'un dispositif selon l'une quelconque des revendications 1 à 11 pour l'hémodialyse, l'hémodiafiltration, ou l'hémofiltration.

**Fig. 1**

**Fig. 2**

**Fig. 3**

**Fig. 4**

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1201293 A1
- US 6271023 B1
- US 2009139925 A1
- US 6214232 B1
- US 4880440 A
- WO 9104758 A1
- WO 200725738 A2